# EUROPEAN PATENT SPECIFICATION

(11) **EP 2 594 189 B1**
(45) Date of publication and mention of the grant of the patent: **27.07.2016**
(21) Application number: 12192816.2
(22) Date of filing: 15.11.2012
(51) Int. Cl.: A61B 1/012, A61B 1/00

(54) **Guide assembly for endoscope and endoscope system**
Führungsanordnung für Endoskop und Endoskopsystem
Ensemble de guidage pour endoscope et système endoscopique

(30) Priority: 18.11.2011 JP 2011252940
(43) Date of publication of application: 22.05.2013
(73) Proprietor: Fujifilm Corporation, Minato-ku Tokyo (JP)
(72) Inventor: Ueda, Yoshihiro, Ashigarakami-gun, Kanagawa (JP)
(74) Representative: Klunker . Schmitt-Nilson . Hirsch

(56) References cited:
- EP-A1- 2 368 482
- EP-A2- 2 016 914
- US-A- 4 346 703
- US-A- 5 487 728

## Description

### BACKGROUND OF THE INVENTION

### 1. Field of the Invention

The present invention relates to a guide assembly for an endoscope and an endoscope system. More particularly, the present invention relates to a guide assembly for an endoscope and an endoscope system, to which a syringe or the like can be easily connected typically at the time of emergency, and in which the connection can be safe in a normal use without errors.

### 2. Description Related to the Prior Art

An endoscope is a widely used medical instrument, of which an elongated tube or insertion tube is entered in a lower GI tract or gastrointestinal tract, for example a large or small intestine, for the purpose of imaging of a body cavity and treatment. The large or small intestine in the lower GI tract is an extremely tortuous organ. Manipulation for entry of the elongated tube in a deep manner is technically difficult, because force of manual operation is difficult to transmit to a tip of the elongated tube during advance of the elongated tube. The use of a guide assembly or overtube assembly (sliding tube) is known in the field. The guide assembly of a tubular shape is mounted on the elongated tube of the endoscope by receiving its entry, so that the endoscope is entered in the body together with the guide assembly. The elongated tube is guided by the guide assembly, so that unwanted flexing and bending of the elongated tube can be prevented during advance of he elongated tube.

JP-A 2004-358222 (corresponding to JP-B 3888359) EP 2016914 B2 and JP-A 11-290263 disclose a guide assembly with a balloon at its distal end. The guide assembly is used together with the endoscope. When the balloon is inflated, the guide assembly is anchored on the wall in the lower GI tract. The elongated tube of the endoscope and the guide assembly are advance alternately with one another by inflating and deflating the balloon, so that the elongated tube can be entered deeply in the lower GI tract.

A flow channel is formed through the guide assembly for supply and suction of fluid in connection with the balloon, such as water or air. One end opening of the flow channel communicates with the inside of the balloon. A second end opening of the flow channel is connected with a receiving port of a balloon control unit. The fluid is supplied and sucked by the balloon control unit connected to the receiving port, so as to inflate and deflate the balloon.

In the guide assembly of JP-A 2004-358222 (corresponding to JP-B 3888359) and JP-A 11-290263, the receiving port communicating with the flow channel is so shaped as to connect with the balloon control unit. However, the receiving port has a somewhat simple shape without consideration of connection with a syringe or the like. It is impossible readily to connect the syringe to the receiving port in an emergency state of an accident of the balloon control unit or the like.

It is conceivable to form the receiving port at the end of the flow channel in a shape compatible to the syringe. However, there is a type of treatment in which the endoscope is used and the syringe is connected with the endoscope for the purpose of supplying a pharmaceutical drug or other fluid to a body part in a body cavity. The compatible shape of the receiving port to the syringe may cause an accident of an improper coupling of the syringe. In this context, US4,366,703 and US5,487,728 disclose known connectors.

Document EP 2368482 discloses a guide assembly with the features of the preamble of claim 1.

### SUMMARY OF THE INVENTION

In view of the foregoing problems, an object of the present invention is to provide a guide assembly for an endoscope and an endoscope system, to which a syringe or the like can be easily connected typically at the time of emergency, and in which the connection can be safe in a normal use without errors.

In order to achieve the above and other objects and advantages of this invention, a guide assembly for an endoscope is defined in claim 1.

Furthermore, a flexible transfer tube is preferably disposed to extend between the proximal end of the flow channel and the coupling sleeve.

The endoscope includes preferably a handle disposed on a proximal side of the elongated tube, the fluid channel extending through the handle. A flow sleeve is disposed on a peripheral surface of the handle in communication with a distal end of the fluid channel, and connectable with the end sleeve.

In another preferred embodiment, the changing means further includes an engagement mechanism for fastening the port adapter on the connector sleeve when in the first mode shape.

In one preferred embodiment, the changing means further comprises an adhesive tape for adhesion of the port adapter to the connector sleeve when in the first mode shape, and for peeling from the port adapter or the coupling sleeve when in the second mode shape.

In still another preferred embodiment, the changing means includes a flexible conversion tube disposed to extend from the proximal end of the flow channel in a proximal direction. The conversion tube includes a proximal tube end portion to which the end sleeve is secured. A tube portion is disposed to extend in a distal direction from the proximal tube end portion, breakable for converting from the first mode shape to the second mode shape, to define the receiving port internally.

Furthermore, visible information is preferably displayed when the changing means is in the second mode shape, for preventing mounting of a device different from the first medical device.

Furthermore, a fluid filter is preferably disposed with the flow channel or the changing means, for preventing liquid from passing the flow channel and allowing gas to pass.

When the changing means converts from the first mode shape to the second mode shape, the fluid filter is preferably removable from the flow channel.

Also, an endoscope system having an endoscope and a guide assembly is provided. The endoscope includes an elongated tube for entry in a body cavity, and an imaging device, disposed at a distal end of the elongated tube, for imaging in the body cavity. The guide assembly includes a tubular housing for mounting on the elongated tube of the endoscope. An inflatable balloon is disposed at a distal end of the tubular housing. A flow channel is formed in the tubular housing, for supply of fluid to the balloon and suction of fluid from the balloon. An end sleeve is disposed at a proximal end of the flow channel, for connection with a selected one of a balloon control device and a fluid channel of the endoscope. A receiving port connects a first medical device of a male tapered shape to the flow channel in place of the balloon control device or the fluid channel of the endoscope. Changing means is convertible from a first mode shape toward a second mode shape, for internally containing the receiving port when in the first mode shape, to set the end sleeve, and for withdrawing the end sleeve by at least partial breakage or elimination when in the second mode shape, to uncover the receiving port.

Therefore, a syringe or the like can be easily connected typically at the time of emergency and safely in a normal use without errors, because of the selective structure between the exclusive modes for the receiving port and the end sleeve.

### BRIEF DESCRIPTION OF THE DRAWINGS

The above objects and advantages of the present invention will become more apparent from the following detailed description when read in connection with the accompanying drawings, in which:
Fig. 1 is a plan illustrating an endoscope system;
Fig. 2 is a front elevation illustrating a tip device of an endoscope;
Fig. 3 is an exploded perspective view illustrating an overtube assembly of a first preferred embodiment;
Fig. 4 is a vertical section illustrating the overtube assembly;
Fig. 5 is a perspective view illustrating a connector of an emergency mode (second mode shape);
Fig. 6 is a perspective view illustrating a connector of a variant of the first embodiment in which a tear line is formed in a port adapter;
Fig. 7 is a vertical section illustrating a connector of another variant having a fluid filter;
Fig. 8 is a plan illustrating another preferred endoscope system of a double balloon type;
Fig. 9 is an explanatory view in a vertical section illustrating the endoscope system;
Fig. 10 is a perspective view illustrating the connector in a normal mode (first mode shape);
Fig. 11 is a vertical section illustrating the connector in the normal mode;
Fig. 12 is a vertical section illustrating another preferred overtube assembly in combination with a breakable tube;
Fig. 13 is an explanatory view in a cross section taken on line XIII-XIII in Fig. 12.

### DETAILED DESCRIPTION OF THE PREFERRED EMBODIMENT(S) OF THE PRESENT INVENTION

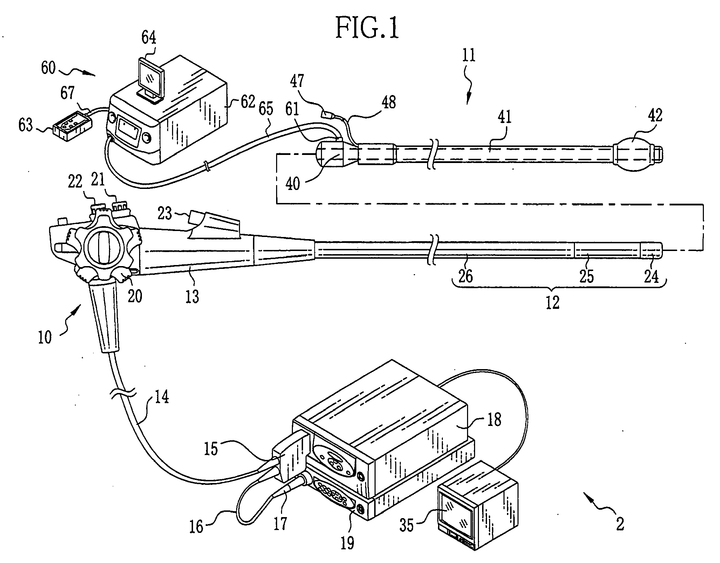

In Fig. 1, an endoscope system 2 includes an electronic endoscope 10 and an overtube assembly 11 or guide assembly for guiding entry of the endoscope 10. The endoscope 10 includes an elongated tube 12 and a handle 13. The elongated tube 12 is entered in a body cavity of a patient, for example, a large intestine of a gastrointestinal tract. The handle 13 is disposed at a proximal end of the elongated tube 12 for manipulation of a doctor or operator. A universal cable 14 is connected to the handle 13. A light source connector 15 is disposed at a tip of the universal cable 14. A cable 16 is a branch of the light source connector 15. A processor connector 17 is disposed at a tip of the cable 16. There is a light source apparatus 18 for which the light source connector 15 is used for connection. For a processing apparatus 19, the processor connector 17 is used for connection.

The handle 13 includes steering wheels 20, a fluid supply button 21 and a suction button 22. The fluid supply button 21 is operable for ejection of air or water through a distal end of the elongated tube 12. An instrument opening 23 is formed in the handle 13 on the side of the elongated tube 12 for receiving entry of a medical instrument for treatment, such as a forceps and electrocautery device.

The elongated tube 12 includes a tip device 24, a steering device 25 and a flexible device 26. The tip device 24 includes a CCD image sensor or the like for imaging an object of interest. The steering device 25 is disposed at a proximal end of the tip device 24, and bendable by operation for steering. The flexible device 26 is disposed at a proximal end of the steering device 25. The overtube assembly 11 can be mounted on the elongated tube 12 in a removable manner. The flexible device 26 has as much a length as several meters for reach of the tip device 24 to an object of interest in the body cavity. The steering device 25 bends up and down and to the right and left in response to operation of the steering wheels 20 at the handle 13. Thus, the tip device 24 can be directed in a desired direction in the body.

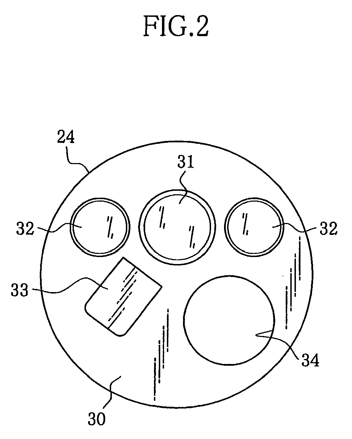

In Fig. 2, the tip device 24 has a distal surface 30. There are an imaging window 31, lighting windows 32, a fluid nozzle spout 33 and an instrument opening 34 disposed on the distal surface 30. The imaging window 31 is disposed at the center of the distal surface 30. The lighting windows 32 are two areas arranged symmetrically with respect to the imaging window 31.

Inside the imaging window 31 is disposed a lens system for receiving image light from the object of interest. The CCD image sensor (or CMOS) is disposed behind the lens system for imaging the object. The image sensor is connected to the processing apparatus 19 by a signal cable extending through the elongated tube 12, the handle 13 and the universal cable 14 toward the processor connector 17. An image of the object received through the imaging window 31 is focused on a light receiving surface of the image sensor, and converted into an image signal. The processing apparatus 19 processes the image signal from the image sensor in various functions of image processing for conversion into a video signal. A display panel 35 is electrically connected, and caused to display an object image. See Fig. 1.

A light guide device guides light from the light source apparatus 18. Distal end portions of the light guide device are disposed behind the lighting windows 32. The light guide device extends through the elongated tube 12, the handle 13 and the universal cable 14 to reach the light source connector 15. A proximal end of the light guide device is disposed within the light source connector 15. Light guided by the light guide device is directed to an object of interest in the body through the lighting windows 32.

The nozzle spout 33, when the fluid supply button 21 is depressed, dispenses air or water toward the imaging window 31 after supply from a fluid supply source incorporated in the light source apparatus 18. An instrument channel (not shown) is formed through the elongated tube 12. The instrument opening 34 is a distal opening of the instrument channel. The distal end of the medical instrument, when entered in the instrument opening 23, becomes protruded through the instrument opening 34.

The overtube assembly 11 includes a grip device 40, a tubular housing 41 or guide tube, and a balloon 42. The grip device 40 is a sleeve formed from plastic material or other rigid material. A connection coupling 51 (connector) for plug-in is disposed on the grip device 40. An example of material for the tubular housing 41 is polyurethane or other flexible material. The tubular housing 41 receives entry of the grip device 40, and is attached to its distal end portion. The overtube assembly 11 is disposable and will be discarded after use.

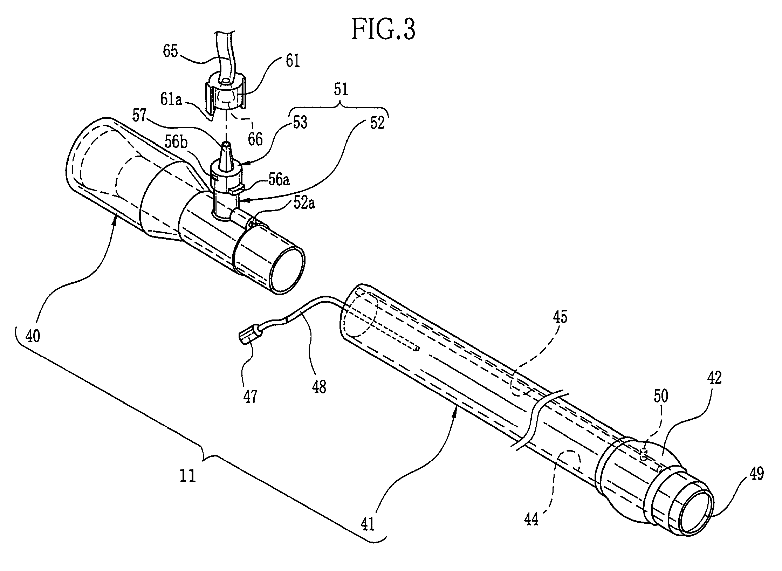

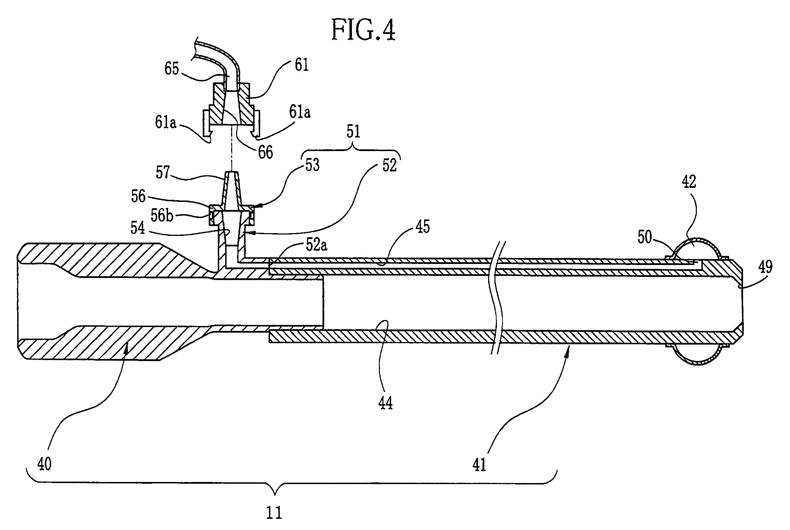

In Figs. 3 and 4, the tubular housing 41 of the overtube assembly 11 includes a tube lumen 44 and a flow channel 45 both extending in the entire length. The tube lumen 44 receives the elongated tube 12 of the endoscope 10, and has an inner diameter slightly larger than an outer diameter of the elongated tube 12. An inner surface of the tube lumen 44 is coated with a hydrophilic coating material (lubricant coating material), such as polyvinylpyrrolidone.

To use the overtube assembly 11, lubricant fluid such as water is supplied on to the inner surface of the tube lumen 44 or between the elongated tube 12 and the tubular housing 41, to lower the friction between those. A connection coupling 47 illustrated in Fig. 1 is used for supply of the lubricant fluid from an injection device (not shown). A small diameter tube 48 extends from the connection coupling 47, and has a distal end coupled to a proximal end of the tube lumen 44. The lubricant fluid injected from the connection coupling 47 flows through the small diameter tube 48 and is supplied to the inner surface of the tube lumen 44.

The tubular housing 41 is formed by working a multi-lumen tube of which a shape of a cross section is constant. A tapered inner surface 49 is formed at a distal end of the tubular housing 41 for decreasing the inner diameter. See Fig. 1. When the elongated tube 12 of the endoscope 10 is advanced into the tube lumen 44, the tapered inner surface 49 decreases an interval between the elongated tube 12 and the distal end of the tubular housing 41, to prevent leaking of the lubricant fluid in a distal direction from the tubular housing 41. The tapered inner surface 49 may be formed on the entire circumference of the end of the tubular housing 41, but may be formed locally therewith.

The balloon 42 is secured to the peripheral surface of the distal end of the tubular housing 41. The balloon 42 is formed from rubber or other elastic material, and shaped in a sleeve shape. The balloon 42 includes a balloon portion at the center and a pair of balloon ends. The balloon 42 is fitted around the distal end portion of the tubular housing 41. A string material is wound about the balloon ends, and sealed with sealant or adhesive agent, so as to fix the balloon 42 to the tubular housing 41.

The flow channel 45 is formed through a wall of the tube lumen 44, and supplies and sucks fluid (air) in connection with the balloon 42. A distal end of the flow channel 45 is positioned at the balloon 42 but does not reach a distal end of the tube lumen 44. An end opening 50 is formed in the peripheral surface of the tubular housing 41 as an open end of the flow channel 45. The end opening 50 is positioned in the balloon 42, and inflates and deflates the balloon 42 by supply and suction of the fluid. A proximal end of the flow channel 45 communicates with the connection coupling 51 at the grip device 40.

The connection coupling 51 includes a coupling sleeve 52 (connector housing) and a port adapter 53 or conversion adapter as changing means fitted on the coupling sleeve 52. The coupling sleeve 52 has an L-shaped conduit, and formed with the grip device 40. One end portion 52a of the coupling sleeve 52 is coupled to the flow channel 45 in a stationary manner. A tapered receiving port 54 or plug-in port for a medical device is formed in a second end portion of the coupling sleeve 52 on a distal side of the grip device 40 in the radial direction. The tapered receiving port 54 is in a female Luer tapered shape, and operable for coupling of a syringe for the emergency by way of a medical device of a male tapered shape.

The port adapter 53 includes an adapter cap 56 and a tapered end sleeve 57 or projection as a normal fluid coupling (for the normal mode). The adapter cap 56 is fitted on the coupling sleeve 52. The tapered end sleeve 57 projects in a male Luer tapered shape. An inner surface of the adapter cap 56 is slightly smaller than an outer surface of the coupling sleeve 52. The adapter cap 56 is tightly fitted on the coupling sleeve 52 to keep the port adapter 53 connected without easy removal. If the port adapter 53 is pulled in the axial direction by manual force of an ordinary person, namely force equal to or more than a predetermined level, the port adapter 53 becomes removed. Note that other structures for keeping the port adapter 53 connected without easy removal from the coupling sleeve 52 can be used. For example, adhesive agent of a small amount can be applied to the inner surface of the adapter cap 56 or the outer surface of the coupling sleeve 52.

A holding projection 56a is formed near to a proximal end of the adapter cap 56, and projects from its peripheral surface. See Fig. 3. The holding projection 56a facilitates grasping of the adapter cap 56, so that the port adapter 53 can be removed easily. Engagement cutouts 56b are formed in the peripheral surface of the adapter cap 56.

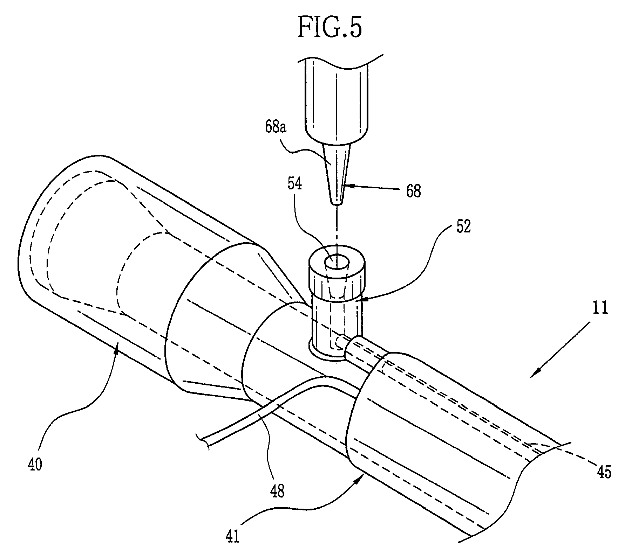

Figs. 3 and 4 illustrate a normal mode of the connection coupling 51 (first mode shape). The adapter cap 56 of the port adapter 53 is fitted on the coupling sleeve 52 to contain the tapered receiving port 54 internally. The tapered end sleeve 57 appears externally. If the port adapter 53 is pulled by force equal to or more than a predetermined level, the port adapter 53 is removed against the tight connection between the adapter cap 56 and the coupling sleeve 52. The tapered receiving port 54 of the coupling sleeve 52 in Fig. 5 appears externally in the emergency mode (second mode shape).

Furthermore, it is preferable to provide visible warning information in the vicinity of the distal end of the coupling sleeve 52 or a portion for fitting the port adapter 53, for example, conspicuous color, indicia and the like. Examples of the conspicuous color are red and yellow. Examples of the indicia are a triangular sign, a star sign and the like. This is effective in preventing improper connection of a tool other than the syringe in an emergency mode of uncovering the tapered receiving port 54, because the conspicuous color or the indicia can notify the operator of the present state.

A connection coupling 61 for plug-in of a balloon control unit 60 is plugged to the connection coupling 51. The balloon control unit 60 supplies and sucks air to inflate and deflate the balloon 42.

In Fig. 1, the balloon control unit 60 operates for supply and suction of fluid such as air, to inflate and deflate the balloon 42, and includes a fluid pumping device 62 or main unit, a switch interface 63 or hand switch for control, and a display panel 64 or balloon monitor. The fluid pumping device 62 includes a pump, sequencer and the like.

The balloon control unit 60 supplies the balloon 42 with fluid for inflation, and controls pressure of the fluid at a regular level to keep the balloon 42 inflated. Also, the balloon control unit 60 sucks fluid from the balloon 42 for deflation, and controls pressure of the fluid at a regular level to keep the balloon 42 deflated.

The display panel 64 displays information of the balloon 42 during the inflation and deflation, such as pressure level, statuses of the inflation and deflation and the like. Note that the display panel 35 can be driven to display the information of the balloon 42 in a manner of superimposition on an object image from the endoscope 10.

The fluid pumping device 62 of the balloon control unit 60 includes a front panel, a power switch, a stop switch and a pressure indicator (not shown). The stop switch on the front panel is a safety switch operable for emergency. The pressure indicator is a panel for indicating a pressure level of the balloon 42, and displays error code if an accident such as breakage of the balloon 42 occurs.

A fluid tube 65 is secured to the front panel of the fluid pumping device 62 for supply and suction of fluid in connection with the balloon 42. A check valve device (not shown) is provided between the fluid tube 65 and the fluid pumping device 62, and is constituted by a fluid filter which is contained in a disk-shaped case for separation of liquid from gas. The case is mounted on the front panel of the fluid pumping device 62 in a removable manner. The check valve device prevents body fluid or other fluid from entering the fluid pumping device 62 even if the balloon 42 is broken.

The connection coupling 61 is disposed at a distal end of the fluid tube 65. The fluid tube 65 can be coupled to the flow channel 45 by plugging the connection coupling 61 to the connection coupling 51 of the overtube assembly 11.

Various switches are provided in the switch interface 63, such as a stop switch, pressure switch and the like. The stop switch is similar to that in the fluid pumping device 62. The pressure switch is operable for pressurizing and depressurizing the balloon 42 on the side of the endoscope. A cable 67 connects the switch interface 63 to the fluid pumping device 62 electrically.

The connection coupling 61 is a sleeve formed from a plastic material. A tapered channel 66 is formed through the connection coupling 61 in a female Luer tapered shape. The tapered end sleeve 57 of the connection coupling 51 can be fitted in the tapered channel 66. The tapered channel 66 is an open end of the fluid tube 65.

Engagement claws 61a project from the peripheral surface of the connection coupling 61, and are engaged with the engagement cutouts 56b of the connection coupling 51 for fastening in a press-fit manner. When the tapered channel 66 of the connection coupling 61 receives entry of the tapered end sleeve 57 of the connection coupling 51, the engagement claws 61a fasten the engagement cutouts 56b. Thus, the connection coupling 61 is plugged to the connection coupling 51 inseparably. The balloon control unit 60 is ready to operate for supply and suction of fluid in connection with the balloon 42.

The operation of the above construction is described now. At first, the overtube assembly 11 is mounted on the elongated tube 12 for use of the endoscope 10. The connection coupling 61 of the balloon control unit 60 is plugged to the connection coupling 51 of the overtube assembly 11. The light source connector 15 and the processor connector 17 are connected to respectively the light source apparatus 18 and the processing apparatus 19.

The doctor or operator enters the elongated tube 12 and the overtube assembly 11 into the body in a push method in an alternately advancing manner. The balloon 42 is inflated by operating the switch interface 63 of the balloon control unit 60 as required, to anchor the overtube assembly 11 in a wall of a body cavity of the body, so that the elongated tube 12 can advance more deeply. Also, the balloon 42 is deflated so as to advance the overtube assembly 11 more deeply. It is possible to image an object by entry of the elongated tube 12 in the body cavity.

In the normal mode described above, the tapered end sleeve 57 of the connection coupling 51 appears externally. The tapered channel 66 of the connection coupling 61 can receive entry of the tapered end sleeve 57 for coupling the flow channel 45 of the overtube assembly 11 to the fluid tube 65 of the balloon control unit 60. If failure of the balloon control unit 60 should occur, the port adapter 53 of the connection coupling 51 can be removed to uncover the tapered receiving port 54 for the emergency mode. Thus, a syringe tip 68a of a syringe 68 in a male Luer tapered shape in Fig. 5 can be coupled to the tapered receiving port 54 for inflation and deflation of the balloon 42. Failure of the balloon control unit 60 can be easily coped with.

A device for use with the endoscope 10 may operate for connection with a syringe. In the connection coupling 51 of the overtube assembly 11, the tapered end sleeve 57 appears externally in the normal mode. The tapered receiving port 54 is contained in the inside of the port adapter 53. This is effective in preventing improper connection of a syringe.

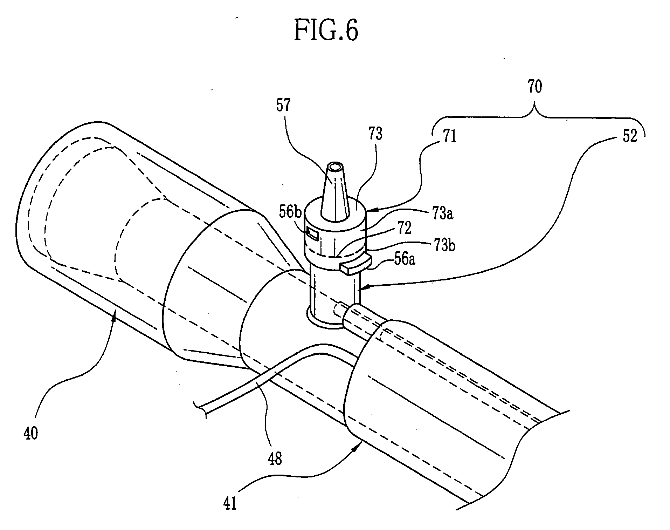

In the above embodiment, the port adapter 53 is entirely removed from the coupling sleeve 52 to uncover the tapered receiving port 54. However, the port adapter 53 can be removed at least partially. In Fig. 6, a variant of a connection coupling 70 for plug-in is illustrated, and includes a port adapter 71 or conversion adapter as changing means. A tear line 72 is formed in the port adapter 71, which can be torn along the tear line 72 and partially removed. The port adapter 71 as a single piece is constituted by an adapter cap 73 and the tapered end sleeve 57.

The adapter cap 73 includes an annular cap portion 73a or distal portion, and a cap skirt 73b or proximal portion. The annular cap portion 73a is mounted on a distal end of the coupling sleeve 52. The cap skirt 73b is fixedly secured to an outer surface of the coupling sleeve 52. The tear line 72 is defined between the annular cap portion 73a and the cap skirt 73b. The adapter cap 73 is tearable along the tear line 72 to separate the annular cap portion 73a from the cap skirt 73b when the annular cap portion 73a is rotated strongly relative to the cap skirt 73b. In the normal mode, the port adapter 71 keeps the tapered receiving port 54 contained internally, and causes the tapered end sleeve 57 to appear externally. The annular cap portion 73a, when rotated by force equal to or higher than a predetermined level, is separated from the cap skirt 73b. The adapter cap 73 can be changed over to the emergency mode in which the tapered receiving port 54 of the coupling sleeve 52 appears externally by removing the annular cap portion 73a.

A structure other than the tear line 72 can be formed for facilitating removal of the annular cap portion 73a in the port adapter 71. For example, a small thickness portion can be formed between the annular cap portion 73a and the cap skirt 73b for facilitating breakage. Such a structure intended for the breakage is effective in notifying the operator of a change of the form from the normal mode to the emergency mode, for example, noise (click) of breakage or manual touch of the adapter cap 73.

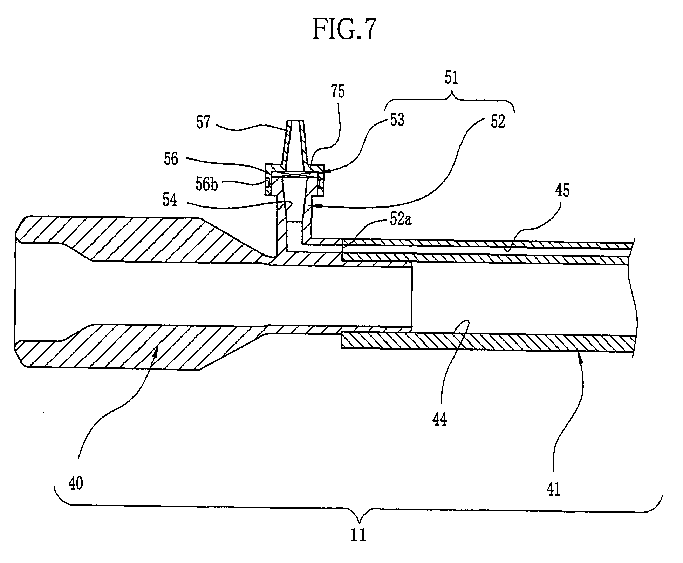

In Fig. 7, a variant of the first embodiment is illustrated. A fluid filter 75 is disposed between the port adapter 53 and the coupling sleeve 52. The fluid filter 75 is squeezed between the adapter cap 56 and the coupling sleeve 52. An example of the fluid filter 75 is the same as the filter for the check valve device in the balloon control unit 60. The fluid filter 75 allows passage of gas but prevents passage of liquid. Thus, liquid can be prevented from flowing into the balloon 42. In the emergency, the fluid filter 75 is removed together with the port adapter 53, so that a syringe can be connected to the tapered receiving port 54 of the coupling sleeve 52. It is easy to operate with the syringe after removing the fluid filter 75, because it is important to remove liquid from the flow channel 45 and the balloon 42. Note that the position of the fluid filter 75 may be determined at any suitable point in a range from the flow channel 45 to the port adapter 53.

In the first embodiment, the holding projection 56a is formed for easy holding of the port adapter 53. However, it is possible to use various forms for facilitating manual holding of the adapter, such as patterned projections on the peripheral surface of the adapter cap, a knurled pattern of projections and recesses on the peripheral surface, an elliptically formed section, and the like.

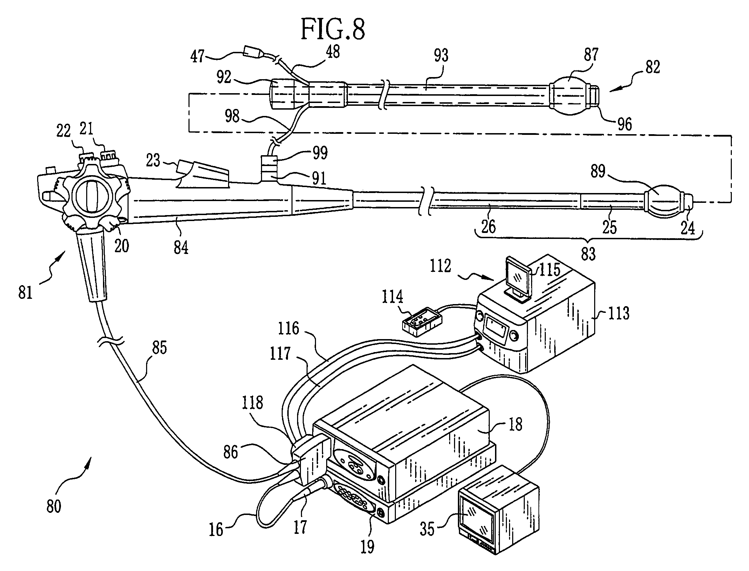

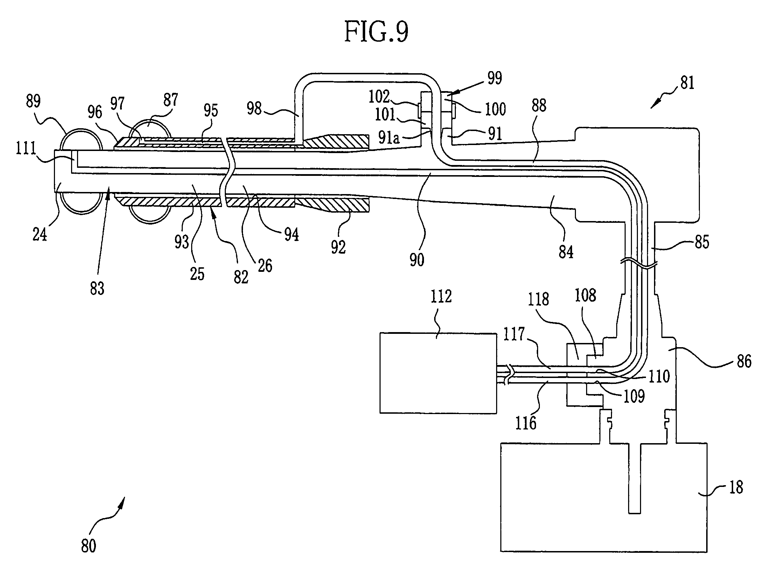

In the above embodiment, the port adapter 53 is for coupling to the connection coupling 61 of the balloon control unit 60. In Figs. 8 and 9, a second preferred endoscope system is illustrated. Changing means is provided in a connection coupling for use with a transfer tube. A flow channel is formed through an endoscope for coupling to a balloon control unit. An endoscope system 80 of the embodiment is a double balloon type, and includes an electronic endoscope 81 with a balloon, and an overtube assembly 82 or guide assembly with a balloon. Elements similar to those of the above embodiment are designated with identical reference numerals.

The endoscope 81 includes an elongated tube 83 and a handle 84. The elongated tube 83 is entered in a large intestine of a gastrointestinal tract of a patient. The handle 84 is formed with a proximal end of the elongated tube 83 for manipulation of a doctor or operator. A universal cable 85 is connected to the handle 84. A light source connector 86 is disposed at a distal end of the universal cable 85. The elongated tube 83 includes the tip device 24, the steering device 25 and the flexible device 26 in the same structures as the first embodiment. The overtube assembly 82 is mounted on the elongated tube 83.

There is a first balloon 87 disposed on the overtube assembly 82. The endoscope 81 has the elements of the endoscope 10 of the first embodiment, and also a first fluid channel 88, a second balloon 89, and a second fluid channel 90. The first fluid channel 88 supplies and sucks fluid in connection with the first balloon 87. The second balloon 89 is disposed on the tip device 24 of the elongated tube 83. The second fluid channel 90 supplies and sucks fluid in connection with the second balloon 89.

The first fluid channel 88 extends through the handle 84, the universal cable 85 and the light source connector 86, and is constituted by a flexible tube. A flow sleeve 91 is disposed on the handle 84 of the endoscope 10.

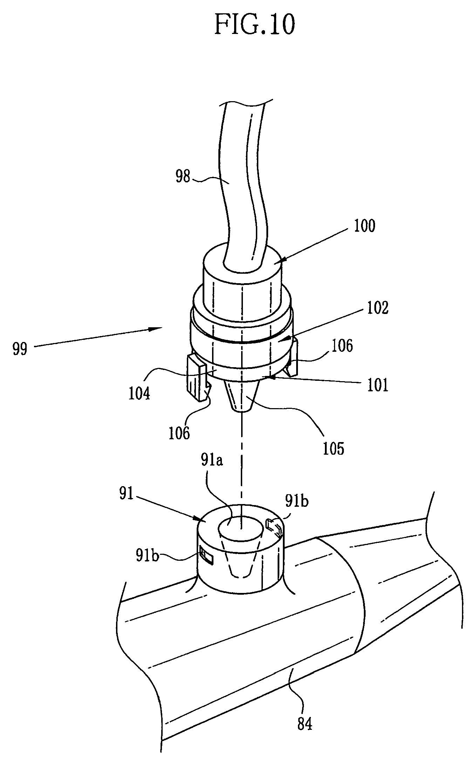

In Fig. 10, the flow sleeve 91 is formed together with a handle housing constituting the handle 84. An example of the material of the flow sleeve 91 is plastic material or metal. A receiving port 91a or plug-in port for a medical device (overtube assembly or guide assembly) is formed in the flow sleeve 91 at the center. A flow channel 95 of the overtube assembly is in communication with the receiving port 91a, which will be described later. The shape of the receiving port 91a is a female Luer tapered shape. Also, an engagement cutout 91b is formed in a peripheral wall of the flow sleeve 91.

The overtube assembly 82 includes a grip device 92, a tubular housing 93 or guide tube, and the first balloon 87. The grip device 92 is formed from plastic material or other rigid material, and is cylindrical. An example of material for the tubular housing 93 is polyurethane or other flexible material. The tubular housing 93 receives entry of the grip device 92, and is attached to its distal end portion. The overtube assembly 82 is disposable and will be discarded after use.

In Fig. 8, the tubular housing 93 has a tube lumen 94, the flow channel 95 and tapered surfaces 96. The tube lumen 94 and the flow channel 95 extend through the tubular housing 93 longitudinally in a manner similar to the tubular housing 41 of the overtube assembly 11 of the first embodiment. The tapered surfaces 96 are disposed at the distal end of the tubular housing 93 to decrease outer and inner diameters.

The first balloon 87 is secured to the peripheral surface of the tubular housing 93. The first balloon 87 of rubber is in a sleeve shape similar to the balloon 42 of the first embodiment. The first balloon 87 is fitted around the distal end of the tubular housing 93 in a fixed manner.

The flow channel 95 is for supply and suction of fluid for the first balloon 87. A first end opening 97 is formed in a peripheral wall of the tubular housing 93 as an open end of the flow channel 95. The first balloon 87 is inflated and deflated by supply and suction of the fluid through the first end opening 97. A transfer tube 98 is coupled to a proximal end of the flow channel 95. A connection coupling 99 for plug-in is coupled to a proximal end of the transfer tube 98. It is preferable for the transfer tube 98 to have a length equal to or larger than a stroke for entry of the elongated tube 83 in the body of the patient. Therefore, the endoscope 81 can be entered without interference with the transfer tube 98.

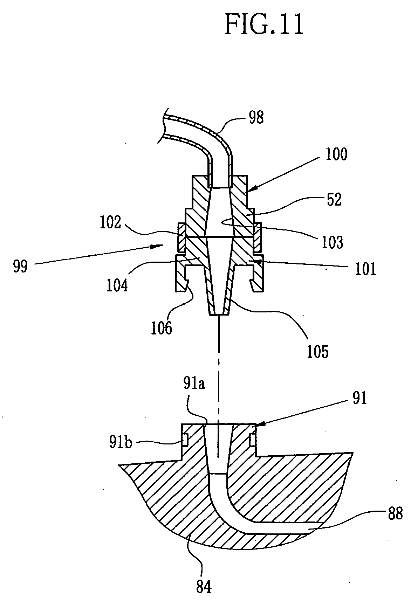

In Figs. 10 and 11, the connection coupling 99 includes a coupling sleeve 100, a port adapter 101 or conversion adapter as changing means, and an adhesive tape 102. The coupling sleeve 100 and the port adapter 101 are plastic parts. The coupling sleeve 100 is attached to one end of the transfer tube 98. A tapered receiving port 103 for a medical device is formed in the coupling sleeve 100 at the center, and is in a female Luer tapered shape for coupling of a syringe for emergency.

The port adapter 101 includes an adapter disk 104, and a tapered end sleeve 105 or projection as a normal fluid coupling (for the normal mode) . The adapter disk 104 has an outer diameter corresponding to the coupling sleeve 100. The tapered end sleeve 105 projects from the adapter disk 104, is hollow at the center, and is formed in a male Luer tapered shape. The adapter disk 104 causes containment of the tapered receiving port 103 or the front surface of the coupling sleeve 100, and also causes contact for continuity of its peripheral surface to the coupling sleeve 100. The adhesive tape 102 is attached to the peripheral surfaces of the coupling sleeve 100 and the adapter disk 104 by extending across their interface, and keeps the adapter disk 104 on the coupling sleeve 100. There is one turn or more of the adhesive tape 102 wound about the peripheral surfaces of the coupling sleeve 100 and the adapter disk 104 for preventing the port adapter 101 from easy removal. When force equal to or higher than a predetermined level is applied to the adhesive tape 102, the adhesive tape 102 can be peeled. Also, an engagement claw 106 for fastening is formed on the adapter disk 104.

In the normal mode (first mode shape) of the connection coupling 99, the port adapter 101 is fitted on the coupling sleeve 100. The tapered receiving port 103 of the coupling sleeve 100 is contained internally, while the tapered end sleeve 105 appears externally. When the port adapter 101 is removed by peeling the adhesive tape 102 strongly, the emergency mode (second mode shape) can be set as the tapered receiving port 103 appears externally.

Furthermore, it is preferable to provide visible warning information in a distal surface of the coupling sleeve 100 or a portion for fitting the port adapter 101, for example, conspicuous color, indicia and the like. This is effective in preventing improper connection of a tool other than the syringe in the emergency mode of uncovering the tapered receiving port 103, because the conspicuous color or the indicia of the coupling sleeve 100 can notify the operator of the present state.

In Fig. 9, a plug-in portion 108 is formed on the light source connector 15. First and second coupling ports 109 and 110 are formed in the plug-in portion 108. A first end of the first fluid channel 88 communicates with the receiving port 91a. A second end of the first fluid channel 88 is the first coupling port 109.

The second balloon 89 is fitted around the tip device 24 of the elongated tube 83. The second balloon 89 is in a sleeve shape and formed from rubber or other elastic material, in a similar manner to the balloon 42 or 87. The second balloon 89 receives entry of the tip device 24, positioned at a predetermined point on the tip device 24, and fixedly fitted thereabout.

The second fluid channel 90 is formed in the endoscope 81, extends through the elongated tube 83, the handle 84, the universal cable 85 and the light source connector 86, and supplies and sucks fluid in connection with the second balloon 89. The second fluid channel 90 is constituted by a flexible tube, of which a distal end is closed at a point of securing a distal end of the second balloon 89. A second end opening 111 is formed in a peripheral surface of the tip device 24, and is an open end of the second fluid channel 90. The second end opening 111 is disposed for the second balloon 89, which is inflated and deflated by supply and suction of fluid through the second end opening 111. The second coupling port 110 is another open end of the second fluid channel 90. A balloon control unit 112 is connected with the first fluid channel 88 and the second fluid channel 90.

The balloon control unit 112 supplies and sucks fluid (air) in connection with each of the first and second balloons 87 and 89 in a discrete manner for alternately inflating those. The balloon control unit 112 includes a fluid pumping device 113 or main unit, a switch interface 114 or hand switch, and a display panel 115 or balloon monitor.

The display panel 115 displays information of the first and second balloons 87 and 89 during their operation of inflation and deflation, such as pressure levels and statuses of the inflation and deflation. The fluid pumping device 113 includes a front panel, a power switch, a stop switch and a pressure indicator (not shown) for the first and second balloons 87 and 89. The stop switch on the front panel is operable for emergency.

Fluid tubes 116 and 117 are disposed to extend from the front panel of the fluid pumping device 113 for supply and suction of fluid in connection with the first and second balloons 87 and 89. A check valve device (not shown) is provided between each of the fluid tubes 116 and 117 and the fluid pumping device 113. One plug-in socket 118 is disposed at distal ends of the fluid tubes 116 and 117. Connection of the plug-in socket 118 to the plug-in portion 108 of the light source connector 86 of the endoscope 81 causes coupling of the fluid tube 116 to the first fluid channel 88 of the endoscope 81 and also coupling of the fluid tube 117 to the second fluid channel 90 of the endoscope 10.

The plug-in socket 118 is plugged to the light source connector 86. The flow channel 95 is coupled to the first fluid channel 88 by the flow sleeve 91 and the connection coupling 99. Thus, the first and second balloons 87 and 89 are ready for operation by control of the balloon control unit 112 to supply and suck fluid.

The operation of the embodiment is described now. For use of the endoscope 10, the overtube assembly 82 is mounted on the elongated tube 83. The tapered end sleeve 105 of the connection coupling 99 is fitted in the receiving port 91a of the flow sleeve 91. The plug-in socket 118 of the balloon control unit 112 is coupled to the light source connector 86.

The doctor or operator enters the elongated tube 83 of the endoscope 81 and the overtube assembly 82 into the body in a push method in an alternately advancing manner. The first balloon 87 is inflated and the second balloon 89 is deflated by operating the switch interface 114 of the balloon control unit 112 as required, to anchor the overtube assembly 82 in a body cavity of the body, so that the elongated tube 83 can advance more deeply. Also, the first balloon 87 is deflated and the second balloon 89 is inflated to anchor the elongated tube 83 in the body cavity, so as to advance the overtube assembly 82 more deeply. It is possible to image an object by entry of the elongated tube 83 in the body cavity.

In the normal mode as described above, the tapered end sleeve 105 of the connection coupling 99 appears externally. The flow channel 95 can be coupled to the first fluid channel 88 of the endoscope 81 by fitting the tapered end sleeve 105 in the receiving port 91a of the flow sleeve 91. If breakage occurs in the balloon control unit 60, the overtube assembly 82 is changed over to the emergency mode by peeling the adhesive tape 102 from the connection coupling 99 and removing the port adapter 101 to uncover the tapered receiving port 103. It is possible to connect a syringe to the tapered receiving port 103. In the normal mode, the tapered receiving port 103 is contained in the inside of the port adapter 101 and the tapered end sleeve 105 appears externally. Improper connection of the syringe can be prevented.

In the second embodiment, the port adapter 101 is entirely removed to uncover the tapered receiving port 103. However, it is possible partially to remove the port adapter 101 to uncover the tapered receiving port 103 in the manner according to the variant of the first embodiment in Fig. 6. Also, the fluid filter 75 can be disposed between the flow channel 95 and the port adapter 101 in a removable manner in the emergency mode of removal of the port adapter 101, which is similar to the first embodiment. Furthermore, various forms for facilitating manual grasping of the port adapter of the second embodiment can be used in the same manner as the first embodiment, such as patterned projections on the peripheral surface of the adapter cap, a knurled pattern of projections and recesses on the peripheral surface, an elliptically formed section, and the like.

In the first embodiment, the port adapter 53 is provided in the connection coupling 51 stationary with the flow channel 45. However, it is possible to provide the adapter in a connector for connection with the flow channel 45 by use of the transfer tube in the manner of the second embodiment. It is preferable for the transfer tube to have a length equal to or larger than a stroke for entry of the elongated tube 12 in the body of the patient.

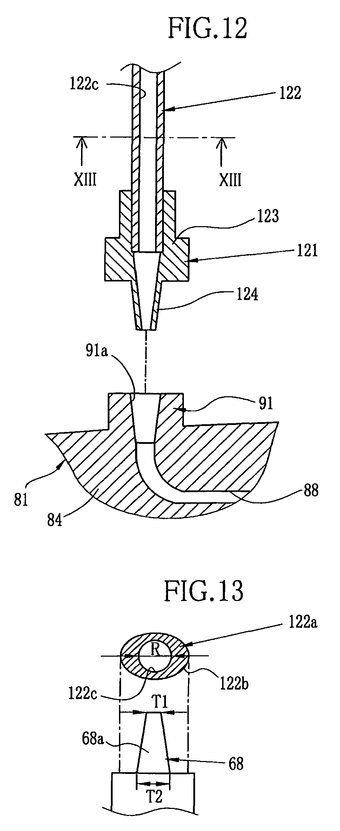

In the second embodiment, the normal mode and the emergency mode are changed over by partially removing the port adapter or conversion adapter. However, it is possible to break a changing means for the purpose of changing over the normal mode and the emergency mode. In Fig. 12, a third preferred embodiment is illustrated. A flexible conversion tube 122 is disposed between a connection coupling 121 for plug-in and a flow channel in an overtube assembly 120. The conversion tube 122 is broken to change over from the normal mode to the emergency mode. For the overtube assembly 120 or guide assembly of the embodiment, the overtube assembly 82 of the second embodiment is repeated but with differences of the conversion tube 122 and the connection coupling 121. The endoscope 81 is repeated, too. The overtube assembly 120 is disposable and will be discarded after use.

An example of the material for the conversion tube 122 is rubber, soft resin or other elastic material. The conversion tube 122 communicates with a proximal end of the flow channel 95. The connection coupling 121 is disposed at the proximal end of the conversion tube 122. The connection coupling 121 includes a proximal tube end portion 123 or coupling sleeve, and a tapered end sleeve 124 or projection formed in a male Luer tapered shape. The conversion tube 122 is shaped in a constant form as viewed in cross sections, irrespective of positions with reference to the axial direction. In Fig. 13, a cross section 122a of a tube portion of the conversion tube 122 is illustrated. An outer surface 122b of the conversion tube 122 is elliptical. An inner surface 122c of the conversion tube 122 is circular. An inner diameter R is equal to the minimum diameter T1 of the syringe tip 68a of the syringe 68, or approximately equal to T1 only with a small difference, and is smaller than the maximum diameter T2 of the same. It is possible to fit the syringe tip 68a of the syringe 68 in the inner surface 122c when the conversion tube 122 is broken.

In the syringe 68 of a commercially available structure, the minimum diameter T1 of the syringe tip 68a is 4 mm or 6 mm. If the minimum diameter T1 is 4 mm, the inner diameter R of the conversion tube 122 is preferably 3-4 mm. If the minimum diameter T1 is 6 mm, the inner diameter R of the conversion tube 122 is preferably 5-10 mm. As the outer surface 122b of the conversion tube 122 is elliptical, the conversion tube 122 can be easily held by a hand of the operator. The conversion tube 122 can be fixedly mounted on a table or the like for the purpose of breaking.

In the normal mode of the conversion tube 122, the tapered end sleeve 124 of the connection coupling 121 appears externally. The inner surface 122c of the conversion tube 122 is contained internally. If the conversion tube 122 is broken in a given position of preference of the operator, the conversion tube 122 can be changed over to cause the inner surface 122c to appear in the emergency mode. In the present embodiment, a position of breaking the conversion tube 122 is not predetermined but can be according to the operator's preference. However, it is possible to assign an indicia to the conversion tube 122 for indicating a position of breaking and break the conversion tube 122 at the indicia. Furthermore, it is preferable to form the conversion tube 122 witch multiple layers in plural colors, and provide visible warning information in a cross section of the conversion tube 122, for example, conspicuous color, indicia and the like. This is effective in preventing improper connection of a tool other than the syringe in the emergency mode of uncovering the inner surface 122c, because the conspicuous color or the indicia can notify the operator of the concurrent state. Also, it is preferable to dispose a fluid filter similar to the fluid filter 75 of the first embodiment in one certain position on the inner surface 122c of the conversion tube 122.

In the above embodiment, the medical device for coupling is the syringe. However, other medical devices of a male tapered shape with a flow channel may be coupled to the tapered receiving port according to the invention, for example, a catheter tip.

In the various embodiments described above, the endoscope system is changed over between the normal mode of the first mode shape and the emergency mode of the second mode shape for the emergency such as breakage of the balloon control unit. However, the first and second mode shapes according to the invention may be predetermined for states other than the normal mode and the emergency mode.

In the first embodiment, the engagement claws 61a of the connection coupling 61 are engageable with the engagement cutouts 56b of the adapter cap 56. However, the adapter cap 56 may have engagement claws. The connection coupling 61 may have engagement cutouts, which can be engaged with the engagement claws for a press-fit connection.

The inner surface of the tapered receiving port 54 or 91a is conical according to the above embodiments, but can be hemispherical. Furthermore, the outer surface of the tapered end sleeve 57, 105 or 124 and the inner surface of the tapered receiving port 54 or 91a can be pyramidal instead of their conical forms.

In the above embodiments, the endoscope is the electronic endoscope having the CCD image sensor as imaging device. However, the endoscope according to the invention may be a type for use with an optical image guide for imaging.

## Claims

1. A guide assembly (11) for an endoscope (10), comprising:
a tubular housing (41) for mounting on an elongated tube of said endoscope;
an inflatable balloon (42) disposed at a distal end of said tubular housing;
a flow channel (45), formed in said tubular housing, for supply of fluid to said balloon and suction of fluid from said balloon;
an end sleeve (57), disposed at a proximal end of said flow channel, for connection with a selected one of a balloon control device and a fluid channel of said endoscope;
a receiving port (54) for connecting a first medical device (68) of a male tapered shape to said flow channel in place of said balloon control device or said fluid channel of said endoscope; **characterized in** changing means (51, 70) including a coupling sleeve (52) and a port adapter (53, 71), convertible from a first mode shape toward a second mode shape, for internally containing said receiving port when in said first mode shape, to set said end sleeve, and for withdrawing said end sleeve by at least partial breakage or elimination of the port adapter when in said second mode shape, to uncover said receiving port, wherein said end sleeve is in a male Luer tapered shape, and said receiving port is in a female Luer tapered shape.

2. A guide assembly (11) as defined in claim 1, **characterized in that** said second mode shape and said first medical device (48) are configured to be used for an emergency mode.

3. A guide assembly (11) as defined in any one of claims 1-2, **characterized in that** said changing means (51, 70) includes:
a coupling sleeve (52), having said receiving port (54), for communicating with said flow channel;
a port adapter (53, 71), including said end sleeve (57), and fitted on said coupling sleeve.

4. A guide assembly (11) as defined in claim 3, **characterized in** further comprising a flexible transfer tube disposed to extend between said proximal end of said flow channel and said coupling sleeve (52).

5. A guide assembly (11) as defined in any one of claims 1-4, **characterized in that** said endoscope includes:
a handle (13) disposed on a proximal side of said elongated tube, said fluid channel extending through said handle;
a flow sleeve (91), disposed on a peripheral surface of said handle in communication with a distal end of said fluid channel, and connectable with said end sleeve.

6. A guide assembly (11) as defined in claim 3, **characterized in that** said changing means (51, 70) further includes an engagement mechanism for fastening said port adapter on said coupling sleeve when in said first mode shape.

7. A guide assembly (11) as defined in claim 3, **characterized in that** said changing means (51, 70) further comprises an adhesive tape for adhesion of said port adapter to said coupling sleeve when in said first mode shape, and for peeling from said port adapter or said coupling sleeve when in said second mode shape.

8. A guide assembly (11) as defined in claim 1 or 2, **characterized in that** said changing means (51, 70) includes a flexible conversion tube (122) disposed to extend from said proximal end of said flow channel in a proximal direction;
wherein said conversion tube includes:
a proximal tube end portion (123) to which said end sleeve (57) is secured;
a tube portion, disposed to extend in a distal direction from said proximal tube end portion, breakable for converting from said first mode shape to said second mode shape, to define said receiving port internally.

9. A guide assembly (11) as defined in any one of claims 1-8, **characterized in** further comprising visible information, displayed when said changing means (51, 70) is in said second mode shape, for preventing mounting of a device different from said first medical device.

10. A guide assembly (11) as defined in any one of claims 1-8, **characterized in** further comprising a fluid filter (75), disposed with said flow channel (45) or said changing means (51, 70), for preventing liquid from passing said flow channel and allowing gas to pass.

11. A guide assembly (11) as defined in claim 10, **characterized in that** when said changing means (51, 70) converts from said first mode shape to said second mode shape, said fluid filter (75) is removable from said flow channel.

12. An endoscope system having an endoscope (10) and a guide assembly (11) according to one of the preceding claims, said endoscope including an elongated tube for entry in a body cavity, and an imaging device, disposed at a distal end of said elongated tube, for imaging in said body cavity.

## Patentansprüche

1. Führungsaufbau (11) für ein Endoskop (10), umfassend:
ein rohrförmiges Gehäuse (41) zum Anbringen auf einem gestreckten Rohr des Endoskops;
einen aufblasbaren Ballon (42), der an einem distalen Ende des rohrförmigen Gehäuses angeordnet ist;
einen Strömungskanal (45), der in dem rohrförmigen Gehäuse ausgebildet ist, zum Zuführen von Fluid zu dem Ballon und zum Absaugen von Fluid von dem Ballon;
eine Endhülse (57), die an einem proximalen Ende des Strömungskanals angeordnet ist, zum selektiven Verbinden mit entweder einer Ballon-Steuervorrichtung oder eines Fluidkanals des Endoskops;
einen Annahmeanschluss (54) zum Verbinden einer ersten medizinischen Vorrichtung (68) mit einer männlichen, abgeschrägten Form mit dem Strömungskanal anstelle der Ballon-Steuervorrichtung oder des Fluidkanals des Endoskops;
**gekennzeichnet durch**
ein Veränderungsmittel (51, 70) umfassend eine Koppelhülse (52) und einen Anschlussadapter (53, 71), das aus einer Form einer ersten Mode zu einer Form einer zweiten Mode konvertierbar ist, zum Aufnehmen des Annahmeanschlusses im Inneren wenn in der Form der ersten Mode, zum Einsetzen der Endhülse, und zum Abnehmen der Endhülse **durch** wenigstens teilweisen Bruch oder Entfernung des Anschlussadapters wenn in der Form der zweiten Mode, zum Abdecken des Annahmeanschlusses, wobei die Endhülse in einer männlichen abgeschrägten Luer-Form vorliegt und der Annahmeanschluss in einer weiblichen abgeschrägten Luer-Form vorliegt.

2. Führungsaufbau (11) nach Anspruch 1, **dadurch gekennzeichnet, dass** die Form der zweiten Mode und die erste medizinische Vorrichtung (48) eingerichtet sind bei einem Notfall-Modus verwendet zu werden.

3. Führungsaufbau (11) nach einem der Ansprüche 1 bis 2, **dadurch gekennzeichnet, dass** das Veränderungsmittel (51, 70) beinhaltet:
eine Koppelhülse (52), die den Annahmeanschluss (54) aufweist, zur Kommunikation mit dem Strömungskanal;
einen Anschlussadapter (53, 71), beinhaltend die Endhülse (57), der auf die Koppelhülse eingepasst ist.

4. Führungsaufbau (11) nach Anspruch 3, **dadurch gekennzeichnet, dass** er weiterhin ein flexibles Transferrohr umfasst, das so angeordnet ist, dass es sich zwischen dem proximalen Ende des Strömungskanals und der Koppelhülse (52) erstreckt.

5. Führungsaufbau (11) nach einem der Ansprüche 1 bis 4, **dadurch gekennzeichnet, dass** das Endoskop beinhaltet:
ein Griffstück (13), das auf einer proximalen Seite des gestreckten Rohrs angeordnet ist, wobei der Strömungskanal sich durch das Griffstück hindurch erstreckt;
eine Strömungshülse (91), die auf einer peripheren Oberfläche des Griffstücks in Kommunikation mit einem distalen Ende des Strömungskanals angeordnet ist und mit der Endhülse verbindbar ist.

6. Führungsaufbau (11) nach Anspruch 3, **dadurch gekennzeichnet, dass** das Veränderungsmittel (51, 71) weiterhin einen Eingriffsmechanismus zum Befestigen des Anschlussadapters auf der Kopplungshülse wenn in der Form der ersten Mode beinhaltet.

7. Führungsaufbau (11) nach Anspruch 3, **dadurch gekennzeichnet, dass** das Veränderungsmittel (51, 70) weiterhin ein Haftband beinhaltet zum Anhaften des Anschlussadapters an der Koppelhülse wenn in der Form der ersten Mode und zum Ablösen von dem Anschlussadapter oder der Koppelhülse wenn in der Form der zweiten Mode.

8. Führungsaufbau (11) nach Anspruch 1 oder 2, **dadurch gekennzeichnet, dass** das Veränderungsmittel (51, 70) ein flexibles Konversionsrohr (122) beinhaltet, dass so angeordnet ist, dass es sich von dem proximalen Ende des Strömungskanals in eine proximale Richtung erstreckt;
wobei das Konversionsrohr beinhaltet:
einen proximalen Rohrendabschnitt (123), an welchem die Endhülse (57) angebracht ist;
einen Rohrabschnitt, der so angeordnet ist, dass er sich in einer distalen Richtung von dem proximalen Rohrendabschnitt erstreckt, der zum Konvertieren von der Form der ersten Mode zu der Form der zweiten Mode zerbrechlich ist, zum Definieren des Inneren des Annahmeanschlusses.

9. Führungsaufbau (11) nach einem der Ansprüche 1 bis 8, **dadurch gekennzeichnet, dass** er weiterhin sichtbare Information umfasst, die angezeigt wird, wenn das Veränderungsmittel (51, 70) in der Form der zweiten Mode ist, zum Verhindern des Befestigens einer Vorrichtung die von der ersten medizinischen Vorrichtung verschieden ist.

10. Führungsaufbau (11) nach einem der Ansprüche 1 bis 8, **dadurch gekennzeichnet, dass** er weiterhin einen Fluidfilter (75) umfasst, der an dem Strömungskanal (45) oder dem Veränderungsmittel (51, 70) angeordnet ist, zum Verhindern, das Flüssigkeit den Strömungskanal passiert und zum Erlauben das Gas passiert.

11. Führungsaufbau (11) nach Anspruch 10, **dadurch gekennzeichnet, dass** wenn das Veränderungsmittel (51, 70) von der Form der ersten Mode zu der Form der zweiten Mode konvertiert, der Fluidfilter (75) aus dem Strömungskanal entnehmbar ist.

12. Endoskopsystem mit einem Endoskop (10) und einem Führungsaufbau (11) nach einem der vorhergehenden Ansprüche, wobei das Endoskop ein gestrecktes Rohr zum Einsetzen in eine Körperkavität und eine Abbildungsvorrichtung, die an einem distalen Ende des gestreckten Rohrs angeordnet ist, zum Abbilden der Körperkavität beinhaltet.

## Revendications

1. Ensemble de guidage (11) destiné à un endoscope (10), comprenant :
un logement tubulaire (41) destiné à un montage sur un tube allongé dudit endoscope ;
un ballonnet gonflable (42) disposé sur une extrémité distale dudit logement tubulaire ;
un canal de flux (45), formé dans ledit logement tubulaire, destiné à une alimentation de fluide vers ledit ballonnet et à une aspiration du fluide à partir du ballonnet ;
un manchon d'extrémité (57), disposé sur une extrémité proximale dudit canal de flux, pour un raccordement avec un dispositif sélectionné parmi un dispositif de contrôle de ballonnet et un canal de fluide dudit endoscope ;
un orifice récepteur (54) destiné à raccorder un premier dispositif médical (68) présentant une forme mâle à amincissement progressif audit canal de flux à la place dudit dispositif de contrôle de ballonnet ou dudit canal de fluide dudit endoscope,
**caractérisé par** un moyen de changement (51, 70) incluant un manchon d'accouplement (52) et un adaptateur d'orifice (53, 71), pouvant être converti d'une forme de premier mode à une forme de second mode, pour contenir de manière interne ledit orifice récepteur lorsque dans ladite forme de premier mode, afin de monter ledit manchon d'extrémité, et pour retirer ledit manchon d'extrémité par au moins une rupture partielle ou une élimination de l'adaptateur d'orifice lorsque dans ladite forme de second mode, afin de découvrir ledit orifice récepteur, dans lequel ledit manchon d'extrémité présente une forme mâle à amincissement progressif de Luer, et ledit orifice récepteur présente une forme femelle à amincissement progressif de Luer.

2. Ensemble de guidage (11) selon la revendication 1, **caractérisé en ce que** ladite forme de second mode et ledit premier dispositif médical (48) sont configurés pour être utilisés pour un mode d'urgence.

3. Ensemble de guidage (11) selon l'une quelconque des revendications 1 à 2, **caractérisé en ce que** ledit moyen de changement (51, 70) inclut :
un manchon d'accouplement (52), présentant ledit orifice récepteur (54) en vue d'une communication avec ledit canal de flux ;
un adaptateur d'orifice (53, 71), incluant ledit manchon d'extrémité (57), et adapté sur ledit manchon d'accouplement.

4. Ensemble de guidage (11) selon la revendication 3, **caractérisé en ce qu'**il comprend en outre un tube de transfert flexible disposé de manière à s'étendre entre ladite extrémité proximale dudit canal de flux et ledit manchon d'accouplement (52).

5. Ensemble de guidage (11) selon l'une quelconque des revendications 1 à 4, **caractérisé en ce que** ledit endoscope inclut :
un manche (13) disposé sur un côté proximal dudit tube allongé, ledit canal de fluide s'étendant à travers ledit manche ;
un manchon de flux (91), disposé sur une surface périphérique dudit manche en communication avec une extrémité distale dudit canal de fluide, et pouvant être raccordé audit manchon d'extrémité.

6. Ensemble de guidage (11) selon la revendication 3, **caractérisé en ce que** ledit moyen de changement (51, 70) inclut en outre un mécanisme de prise destiné à attacher ledit adaptateur d'orifice sur ledit manchon d'accouplement lorsque dans ladite forme de premier mode.

7. Ensemble de guidage (11) selon la revendication 3, **caractérisé en ce que** ledit moyen de changement (51, 70) comprend en outre un ruban adhésif destiné à l'adhérence dudit adaptateur d'orifice audit manchon d'accouplement lorsque dans ladite forme de premier mode, et au décollage du dudit adaptateur d'orifice ou dudit manchon d'accouplement lorsque dans ladite forme de second mode.

8. Ensemble de guidage (11) selon la revendication 1 ou 2, **caractérisé en ce que** ledit moyen de changement (51, 70) inclut en outre un tube de conversion flexible (122) disposé de manière à s'étendre de ladite extrémité proximale dudit canal de flux dans une direction proximale ;
dans lequel ledit tube de conversion inclut :
une partie d'extrémité de tube proximale (123) sur laquelle ledit manchon d'extrémité (57) est fixé ;
une partie de tube, disposée de manière à s'étendre dans une direction distale à partir de ladite partie d'extrémité de tube proximale, pouvant être rompue pour une conversion de ladite forme de premier mode à ladite forme de second mode, afin de définir ledit orifice récepteur de manière interne.

9. Ensemble de guidage (11) selon l'une quelconque des revendications 1 à 8, **caractérisé en ce qu'**il comprend en outre des informations visibles, affichées lorsque ledit moyen de changement (51,70) se trouve dans ladite forme de second mode, pour empêcher le montage d'un dispositif différent dudit premier dispositif médical.

10. Ensemble de guidage (11) selon l'une quelconque des revendications 1 à 8, **caractérisé en ce qu'**il comprend en outre un filtre à fluide (75), disposé avec ledit canal de flux (45) ou ledit moyen de changement (51, 70), pour empêcher le passage de liquide par ledit canal de flux et pour permettre le passage de gaz.

11. Ensemble de guidage (11) selon la revendication 10, **caractérisé en ce que** lorsque ledit moyen de changement (51, 70) convertit de ladite forme de premier mode à la forme de second mode, ledit filtre à fluide (75) peut être retiré dudit canal de flux.

12. Système endoscopique présentant un endoscope (10) et un ensemble de guidage (11) selon l'une quelconque des revendications précédentes, ledit endoscope incluant un tube allongé pour une entrée dans une cavité corporelle, et un dispositif d'imagerie, disposé sur une extrémité distale dudit tube allongé, en vue d'une imagerie dans ladite cavité corporelle.
